(19) Europäisches Patentamt / European Patent Office / Office européen des brevets

(11) **EP 2 672 966 B1**

(12) # EUROPEAN PATENT SPECIFICATION

(45) Date of publication and mention of the grant of the patent: **07.12.2016 Bulletin 2016/49**

(21) Application number: **12702288.7**

(22) Date of filing: **08.02.2012**

(51) Int Cl.: ***A61K 31/34*** *(2006.01)* ***A61P 3/10*** *(2006.01)*

(86) International application number: **PCT/EP2012/052108**

(87) International publication number: **WO 2012/107476 (16.08.2012 Gazette 2012/33)**

(54) **PHARMACEUTICAL COMPOSITION, METHODS FOR TREATING AND USES THEREOF**

PHARMAZEUTISCHE ZUSAMMENSETZUNG, VERFAHREN ZUR BEHANDLUNG UND VERWENDUNG

COMPOSITION PHARMACEUTIQUE, MÉTHODES DE TRAITEMENT ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.02.2011 EP 11153789**

(43) Date of publication of application: **18.12.2013 Bulletin 2013/51**

(73) Proprietor: **Boehringer Ingelheim International GmbH**
**55216 Ingelheim am Rhein (DE)**

(72) Inventors:
- **KLEIN, Thomas**
  **55216 Ingelheim am Rhein (DE)**
- **GREMPLER, Rolf**
  **55216 Ingelheim am Rhein (DE)**
- **MARK, Michael**
  **55216 Ingelheim am Rhein (DE)**

(74) Representative: **Simon, Elke Anna Maria et al**
**Boehringer Ingelheim GmbH**
**Binger Strasse 173**
**55216 Ingelheim am Rhein (DE)**

(56) References cited:
**WO-A1-2010/092125** **WO-A2-2011/060290**

- **PIYA M K; TAHRANI A A; BARNETT A H: "Emerging treatment options for type 2 diabetes", BRITISH JOURNAL OF CLINICAL PHARMACOLOGY, vol. 70, no. 5, 2010, pages 631-644, XP002670159,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 672 966 B1

## Description

### Technical Field of the Invention

The invention relates to a pharmaceutical composition comprising an SGLT2-inhibitor and an GLP-1 receptor agonist as described hereinafter which is suitable in the treatment or prevention of one or more conditions selected from type 2 diabetes mellitus, impaired glucose tolerance, impaired fasting blood glucose, hyperglycemia and other conditions.

Furthermore the invention relates to methods

- for treating diabetes mellitus;
- for treating diabetes mellitus, where treatment with insulin or a GLP-1 receptor agonist is required;
- for preventing, slowing progression of, delaying or treating of a condition or disorder selected from the group consisting of complications of diabetes mellitus;
- for preventing, slowing progression of, delaying, or treating a metabolic disorder;
- for improving glycemic control and/or for reducing of fasting plasma glucose, of postprandial plasma glucose and/or of glycosylated hemoglobin HbA1c;
- for preventing, slowing, delaying or reversing progression from impaired glucose tolerance, impaired fasting blood glucose, insulin resistance and/or from metabolic syndrome to type 2 diabetes mellitus;
- for reducing body weight and/or body fat or preventing an increase in body weight and/or body fat or facilitating a reduction in body weight and/or body fat;
- for preventing, slowing, delaying or treating diseases or conditions attributed to an abnormal accumulation of ectopic fat;
- maintaining and/or improving the insulin sensitivity and/or for treating or preventing hyperinsulinemia and/or insulin resistance,
- for preventing, slowing progression of, delaying, or treating new onset diabetes after transplantation (NODAT) and/or post-transplant metabolic syndrome (PTMS);
- for preventing, delaying, or reducing NODAT and/or PTMS associated complications including micro- and macrovascular diseases and events, graft rejection, infection, and death;
- for treating hyperuricemia and hyperuricemia associated conditions;
- for treating or preventing kidney stones;
- for treating hyponatremia;

in patients in need thereof characterized in that a GLP-1 receptor agonist and a SGLT2 inhibitor are administered in combination or alternation or sequentially.

In addition the present invention relates to the use of an SGLT2 inhibitor for the manufacture of a medicament for use in a method as described hereinbefore and hereinafter.

In addition, the present invention relates to the use of a GLP-1 receptor agonist for the manufacture of a medicament for use in a method as described hereinbefore and hereinafter.

The invention also relates to a pharmaceutical composition according to this invention for use in a method as described hereinbefore and hereinafter.

The invention also relates to a use of a pharmaceutical composition according to this invention for the manufacture of a medicament for use in a method as described hereinbefore and hereinafter.

### Background of the Invention

Type 2 diabetes is an increasingly prevalent disease that due to a high frequency of complications associated with a reduction in life expectancy. Because of diabetes-associated microvascular complications, type 2 diabetes is currently the most frequent cause of adult-onset loss of vision, renal failure, and amputations in the industrialized world. In addition, the presence of type 2 diabetes is associated with a two to five fold increase in cardiovascular disease risk.

After long duration of disease, most patients with type 2 diabetes will eventually fail on oral therapy and become insulin dependent with the necessity for daily injections and multiple daily glucose measurements.

The UKPDS (United Kingdom Prospective Diabetes Study) demonstrated that intensive treatment with metformin, sulfonylureas or insulin resulted in only a limited improvement of glycemic control (difference in HbA1c ~0.9%) as compared to conventional treatment. In addition, even in patients within the intensive treatment arm glycemic control deteriorated significantly over time and this was attributed to deterioration of beta-cell function. Of importance however, despite this deterioration of beta-cell function, was that intensive glycemic treatment was associated with microvascular benefits in the short term (6 years) and macro-mascular benefits in the long term (15 years). Similar phenomenon has also been demonstrated in patients with type 1 diabetes mellitus, e.g. in the diabetes control and complications trial

(DCCT) where a difference in the median HbA1c (~1.9%) between the conventional therapy and intensive therapy group during 6.5 years of study, led to significant relative risk reductions for microvascular complications whereas macrovascular benefits was noted 11 years after the DCCT, e.g. as reported in the EDIC (Epidemiology of Diabetes Interventions and Complications) study, where a relative HbA1c reduction by 10% in one patient compared to another was associated with a hazard ratio of 0.80 for cardiovascular complications. Despite such convincing long term effects of glycemic management many patients with type 2 diabetes or type 1 diabetes remain inadequately treated, partly because of limitations in long term efficacy, tolerability and dosing inconvenience of existing antihyperglycemic therapies.

The high incidence of therapeutic failure might be a major contributor to the high rate of long-term hyperglycemia-associated complications or chronic damages (including micro- and macrovascular complications such as e.g. diabetic nephrophathy, retinopathy or neuropathy, or cardiovascular complications) in patients with type 2.

Therefore, there is an unmet medical need for methods, medicaments and pharmaceutical compositions with a good efficacy with regard to glycemic control, with regard to disease-modifying properties and with regard to reduction of cardiovascular morbidity and mortality while at the same time showing an improved safety profile.

Furthermore, diabetes (particularly type 2 diabetes) is often coexistent and interrelated with obesity and these two conditions together impose a particularly complex therapeutic challenge. Because of the effects of obesity on insulin resistance, weight loss and its maintainance is an important therapeutic objective in overweight or obese individuals with prediabetes, metabolic syndrome or diabetes. Studies have been demonstrated that weight reduction in subjects with type 2 diabetes is associated with descreased insulin resistance, improved measures of glycemia and lipemia, and reduced blood pressure. Maintainance of weight reduction over longer term is considered to improve glycemic control and prevent diabetic complications (e.g. reduction of risk for cardiovascular diseases or events). Thus, weight loss is recommended for all overweight or obese indivuduals who have or are at risk for diabetes. However, obese patients with type 2 diabetes have much greater difficulty losing weight and maintain the reduced weight than the general non-diabetic population.

Therefore it remains a need in the art to provide efficacious, safe and tolerable antidiabetic therapies, particularly for obese or overweight diabetes patients. Within the management of the dual epidemic of type 2 diabetes and obesity ("diabesity"), it is an objective to find therapies which are safe, tolerable and effective in the treatment or prevention of these conditions together, particularly in achieving long term weight reduction and improving glycemic control.

Oral antidiabetic drugs conventionally used in therapy (such as e.g. first- or second-line, and/or mono- or (initial or add-on) combination therapy) include, without being restricted thereto, metformin, sulphonylureas, thiazolidinediones, glinides and $\alpha$-glucosidase inhibitors. A side effect of some of such oral antidiabetics is an unwanted increase of the body weight.

Non-oral (typically injected) antidiabetic drugs conventionally used in therapy (such as e.g. first- or second-line, and/or mono- or (initial or add-on) combination therapy) include, without being restricted thereto, GLP-1 or GLP-1 analogues, and insulin or insulin analogues. An insulin therapy may result in an increase of the body weight of the patient.

Glucagon-like peptide-1 (GLP-1) is a hormon secreted from enteroendocrine L cells of the intestine in response to food. The action of GLP-1 is mediated through the GLP-1 receptor (GLP-1 R). Exogenous GLP-1 administration at pharmacological doses results in effects that are beneficial for treating type 2 diabetes. However, native GLP-1 is subject to rapid enzymatic degradation. For example the medicament exendin-4 is a biotechnologically manufactured GLP-1 analogue with a slower degradation than native GLP-1. A positive side effect in the treatment with exendin-4 is a reduction of the body weight. A discontinuation of a therapy with exendin-4 leads to an increase of the body-weight again.

SGLT2 inhibitors inhibitors represent a novel class of agents that are being developed for the treatment or improvement in glycemic control in patients with type 2 diabetes. Glucopyranosyl-substituted benzene derivative are described in the prior art as SGLT2 inhibitors, for example in WO 01/27128, WO 03/099836, WO 2005/092877, WO 2006/034489, WO 2006/064033, WO 2006/117359, WO 2006/117360, WO 2007/025943, WO 2007/028814, WO 2007/031548, WO 2007/093610, WO 2007/128749, WO 2008/049923, WO 2008/055870, WO 2008/055940. The glucopyranosyl-substituted benzene derivatives are proposed as inducers of urinary sugar excretion and as medicaments in the treatment of diabetes.

Renal filtration and reuptake of glucose contributes, among other mechanisms, to the steady state plasma glucose concentration and can therefore serve as an antidiabetic target. Reuptake of filtered glucose across epithelial cells of the kidney proceeds via sodium-dependent glucose cotransporters (SGLTs) located in the brush-border membranes in the tubuli along the sodium gradient. There are at least 3 SGLT isoforms that differ in their expression pattern as well as in their physico-chemical properties. SGLT2 is exclusively expressed in the kidney, whereas SGLT1 is expressed additionally in other tissues like intestine, colon, skeletal and cardiac muscle. Under normoglycemia, glucose is completely reabsorbed by SGLTs in the kidney, whereas the reuptake capacity of the kidney is saturated at glucose concentrations higher than 10mM, resulting in glucosuria (hence the notion "diabetes mellitus"). This threshold concentration can be decreased by SGLT2-inhibition. It has been shown in experiments with the SGLT inhibitor phlorizin that SGLT-inhibition will partially inhibit the reuptake of glucose from the glomerular filtrate into the blood leading to a decrease in blood glucose concentrations and to glucosuria.

**Aim of the present invention**

The aim of the present invention is to provide a pharmaceutical composition and method for preventing, slowing progression of, delaying or treating a metabolic disorder, in particular of diabetes mellitus and complications of diabetes mellitus.

Another aim of the present invention is to provide a pharmaceutical composition and method for treating patients with type 2 diabetes mellitus.

A further aim of the present invention is to provide a pharmaceutical composition and method for improving glycemic control in a patient in need thereof, in particular in patients with type 2 diabetes mellitus.

Another aim of the present invention is to provide a pharmaceutical composition and method for improving glycemic control in a patient.

Another aim of the present invention is to provide a pharmaceutical composition and method for prolonging the duration of efficacy of a GLP-1 receptor agonist administered to a patient.

Another aim of the present invention is to provide a pharmaceutical composition and method for reducing the required GLP-1 receptor agonist dose in a patient.

Another aim of the present invention is to provide a pharmaceutical composition and method for preventing, slowing or delaying progression from impaired glucose tolerance (IGT), impaired fasting blood glucose (IFG), insulin resistance and/or metabolic syndrome to type 2 diabetes mellitus.

Yet another aim of the present invention is to provide a pharmaceutical composition and method for preventing, slowing progression of, delaying or treating of a condition or disorder from the group consisting of complications of diabetes mellitus, in particular type 2 diabetes mellitus.

A further aim of the present invention is to provide a pharmaceutical composition and method for reducing the weight or preventing an increase of the weight in a patient in need thereof.

A further aim of the present invention is to provide a pharmaceutical composition and method for maintaining the weight, preventing an increase of the weight or reducing the amount and/or rate of weight gain in a patient in need thereof after termination, discontinuation or interruption of a treatment with a GLP1-receptor agonist.

Another aim of the present invention is to provide a new pharmaceutical composition with a high efficacy for the treatment of metabolic disorders, in particular of diabetes mellitus, impaired glucose tolerance (IGT), impaired fasting blood glucose (IFG), and/or hyperglycemia, which has good to very good pharmacological and/or pharmacokinetic and/or physicochemical properties.

Further aims of the present invention become apparent to the one skilled in the art by description hereinbefore and in the following and by the examples.

**Summary of the Invention**

Within the scope of the present invention it has now been found that a combination of a SGLT2 inhibitor and a GLP-1 receptor agonist as defined herein as well as pharmaceutical combinations, compositions or combined or sequential uses according to this invention of a SGLT2 inhibitor and a GLP-1 receptor agonist as defined herein have unexpected and particularly advantageous properties, which make them suitable for the purpose of this invention and/or for fulfilling one or more of above needs. In particular it has surprisingly been found that a combination of a SGLT2 inhibitor and a GLP-1 receptor agonist leads to a higher blood glucose lowering compared with a treatment using the GLP-1 receptor agonist or the SGLT2 inhibitor alone. Thus in order to achieve a certain level of baseline blood glucose the dose of the GLP-1 receptor agonist may be reduced by using a combination of a SGLT2 inhibitor and a GLP-1 receptor agonist. Furthermore an administration of a SGLT2 inhibitor may prolong the lowering of the blood glucose compared with an administration of the GLP-1 receptor agonist alone.

Therefore a combination of a SGLT2 inhibitor and a GLP-1 receptor agonist can advantageously be used for preventing, slowing progression of, delaying or treating a metabolic disorder, in particular for improving glycemic control in patients. This opens up new therapeutic possibilities in the treatment and prevention of type 2 diabetes mellitus, complications of diabetes mellitus and of neighboring disease states.

Therefore, in a first aspect the present invention provides a pharmaceutical composition comprising

(a) an SGLT2 inhibitor, and
(b) a GLP-1 receptor agonist.

According to another aspect of the invention, there is provided a method for treating diabetes mellitus in a patient characterized in that a GLP-1 receptor agonist and an SGLT2 inhibitor are administered, for example in combination or alternation or sequentially, to the patient.

According to another aspect of the invention, there is provided a method for preventing, slowing the progression

of, delaying or treating of a condition or disorder selected from the group consisting of complications of diabetes mellitus such as cataracts and micro- and macrovascular diseases, such as nephropathy, retinopathy, neuropathy, tissue ischaemia, diabetic foot, arteriosclerosis, myocardial infarction, accute coronary syndrome, unstable angina pectoris, stable angina pectoris, stroke, peripheral arterial occlusive disease, cardiomyopathy, heart failure, heart rhythm disorders and vascular restenosis, in a patient in need thereof characterized in that a GLP-1 receptor agonist and an SGLT2 inhibitor are administered, for example in combination or alternation or sequentially, to the patient. In particular one or more aspects of diabetic nephropathy such as hyperperfusion, proteinuria and albuminuria may be treated, their progression slowed or their onset delayed or prevented. The term "tissue ischaemia" particularly comprises diabetic macroangiopathy, diabetic microangiopathy, impaired wound healing and diabetic ulcer. The terms "micro- and macrovascular diseases" and "micro- and macrovascular complications" are used interchangeably in this application.

According to another aspect of the invention, there is provided a method for preventing, slowing the progression of, delaying or treating a metabolic disorder selected from the group consisting of type 2 diabetes mellitus, impaired glucose tolerance (IGT), impaired fasting blood glucose (IFG), hyperglycemia, postprandial hyperglycemia, overweight, obesity, metabolic syndrome, gestational diabetes and diabetes related to cystic fibrosis in a patient in need thereof characterized in that a GLP-1 receptor agonist and an SGLT2 inhibitor are administered, for example in combination or alternation or sequentially, to the patient.

According to another aspect of the invention, there is provided a method for improving glycemic control and/or for reducing of fasting plasma glucose, of postprandial plasma glucose and/or of glycosylated hemoglobin HbA1c in a patient in need thereof characterized in that a GLP-1 receptor agonist and a SGLT2 inhibitor are administered, for example in combination or alternation or sequentially, to the patient.

The pharmaceutical composition according to this invention may also have valuable disease-modifying properties with respect to diseases or conditions related to impaired glucose tolerance (IGT), impaired fasting blood glucose (IFG), insulin resistance and/or metabolic syndrome.

According to another aspect of the invention, there is provided a method for preventing, slowing, delaying or reversing progression from impaired glucose tolerance (IGT), impaired fasting blood glucose (IFG), insulin resistance and/or from metabolic syndrome to type 2 diabetes mellitus in a patient in need thereof characterized in that a GLP-1 receptor agonist and a SGLT2 inhibitor are administered, for example in combination or alternation or sequentially, to the patient.

As by the use of a pharmaceutical composition according to this invention, an improvement of the glycemic control in patients in need thereof is obtainable, also those conditions and/or diseases related to or caused by an increased blood glucose level may be treated.

By the administration of a pharmaceutical composition according to this invention and due to the activity of the SGLT2 inhibitor excessive blood glucose levels are not converted to insoluble storage forms, like fat, but excreted through the urine of the patient. In animal models using a SGLT2 inhibitor it can be seen that loss of fat accounts for the majority of the observed weight loss whereas no significant changes in body water or protein content are observed. Therefore, no gain in weight or even a reduction in body weight is the result.

According to another aspect of the invention, there is provided a method for reducing body weight and/or body fat or preventing an increase in body weight and/or body fat or facilitating a reduction in body weight and/or body fat in a patient in need thereof characterized in that a GLP-1 receptor agonist and a SGLT2 inhibitor are administered, for example in combination or alternation or sequentially, to the patient.

By the administration of a combination or pharmaceutical composition according to the present invention, an abnormal accumulation of ectopic fat, in particular of the liver, may be reduced or inhibited. Therefore, according to another aspect of the present invention, there is provided a method for preventing, slowing, delaying or treating diseases or conditions attributed to an abnormal accumulation of ectopic fat, in particular of the liver, in a patient in need thereof characterized in that a GLP-1 receptor agonist and an SGLT2 inhibitor are administered, for example in combination or alternation or sequentially, to the patient. Diseases or conditions which are attributed to an abnormal accumulation of liver fat are particularly selected from the group consisting of general fatty liver, non-alcoholic fatty liver (NAFL), non-alcoholic steatohepatitis (NASH), hyperalimentation-induced fatty liver, diabetic fatty liver, alcoholic-induced fatty liver or toxic fatty liver.

Usually a therapy with a GLP-1 receptor agonist is associated with a loss of body weight. Such a weight reduction is of particular advantage for diabetic patients being overweight or obese. After termination or discontinuation of said therapy the body weight usually increases again. Said increase may be prevented or attenuated by a therapy with a SGLT2 inhibitor which follows the therapy with a GLP-1 receptor agonist. Therefore another aspect of the invention provides a method for weight reduction, a method for reduction of body fat, a method for preventing an increase of body weight or a method for attenuating an increase of body weight comprising an administration of a GLP-1 receptor agonist in a patient in need thereof followed by an administration of a SGLT2 inhibitor in said patient. The patient may be diagnosed of having diabetes, in particular diabetes mellitus type 2, and the patient may be diagnosed of being overweight or obese. Alternatively the patient is diagnosed of being overweight or obese, but is not diagnosed of having diabetes.

Furthermore, the present invention provides a method for reducing and maintaining body weight in a patient in need thereof (particularly type 2 diabetes patient being obese or overweight) comprising administering one or more effective amounts of a GLP-1 receptor agonist to the patient in a first time period followed by administering one or more effective amounts of a SGLT2 inhibitor to the patient in a second time period. In the first time period a considerable weight loss may be achieved with a therapy using the GLP-1 receptor agonist. The therapy with the SGLT2 inhibitor in the second time period which follows the first time period may replace the therapy with a GLP-1 receptor agonist. In the second time period the glycemic control and the weight control is achieved with the advantageous properties of SGLT2 inhibitor.

Moreover, the present invention provides a SGLT2 inhibitor for body weight reduction, for reduction of body fat, for preventing an increase of body weight or for attenuating an increase of body weight wherein the administration of the SGLT2 inhibitor follows a termination or discontinuation of a treatment with a GLP-1 receptor agonist.

Furthermore, the present invention provides a method for treating a metabolic disorder, in particular of diabetes mellitus and/or complications associated with diabetes mellitus, in a patient who is diagnosed of being overweight or obese, characterized by a first therapy comprising the administration of a GLP-1 receptor agonist to the patient followed by a second therapy comprising the administration of a SGLT2 inhibitor to the patient. The first and/or second therapy may include the administration of one or more further medicaments, for example antidiabetic agents.

Another aspect of the invention provides a method for maintaining and/or improving the insulin sensitivity and/or for treating or preventing hyperinsulinemia and/or insulin resistance in a patient in need thereof characterized in that a GLP-1 receptor agonist and an SGLT2 inhibitor are administered, for example in combination or alternation or sequentially, to the patient.

According to another aspect of the invention, there is provided a method for preventing, slowing progression of, delaying, or treating new onset diabetes after transplantation (NODAT) and/or post-transplant metabolic syndrome (PTMS) in a patient in need thereof characterized in that a GLP-1 receptor agonist and an SGLT2 inhibitor are administered, for example in combination or alternation or sequentially, to the patient.

According to a further aspect of the invention, there is provided a method for preventing, delaying, or reducing NODAT and/or PTMS associated complications including micro- and macrovascular diseases and events, graft rejection, infection, and death in a patient in need thereof characterized in that a GLP-1 receptor agonist and an SGLT2 inhibitor are administered, for example in combination or alternation or sequentially, to the patient.

The pharmaceutical composition according to the invention is capable of facilitating the lowering of serum total urate levels in the patient. Therefore according to another aspect of the invention, there is provided a method for treating hyperuricemia and hyperuricemia-associated conditions, such as for example gout, hypertension and renal failure, in a patient in need thereof characterized in that an a GLP-1 receptor agonist and SGLT2 inhibitor are administered, for example in combination or alternation or sequentially, to the patient.

The administration of a pharmaceutical composition increases the urine excretion of glucose. This increase in osmotic excretion and water release and the lowering of urate levels are beneficial as a treatment or prevention for kidney stones. Therefore in a further aspect of the invention, there is provided a method for treating or preventing kidney stones in a patient in need thereof characterized in that a GLP-1 receptor agonist and an SGLT2 inhibitor are administered, for example in combination or alternation or sequentially, to the patient.

According to a further aspect of the invention, there is provided a method for treating hyponatremia, water retention and water intoxication in a patient in need thereof characterized in that a GLP-1 receptor agonist and an SGLT2 inhibitor are administered, for example in combination or alternation or sequentially, to the patient. By the administration of the pharmaceutical composition according to this invention it may be possible to reverse the effects of hyponatremia, water retention and water intoxication by acting on the kidney to reverse water retention and electrolyte imbalances associated with these diseases and disorders.

According to another aspect of the invention there is provided the use of an SGLT2 inhibitor for the manufacture of a medicament for

- treating diabetes mellitus;
- preventing, slowing progression of, delaying or treating of a condition or disorder selected from the group consisting of complications of diabetes mellitus;
- preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity, metabolic syndrome and gestational diabetes; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance (IGT), impaired fasting blood glucose (IFG), insulin resistance and/or from metabolic syndrome to type 2 diabetes mellitus; or

- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, arteriosclerosis, myocardial infarction, stroke and peripheral arterial occlusive disease; or
- reducing body weight and/or body fat or preventing an increase in body weight and/or body fat or facilitating a reduction in body weight and/or body fat; or
- preventing, slowing, delaying or treating diseases or conditions attributed to an abnormal accumulation of ectopic fat; or
- maintaining and/or improving the insulin sensitivity and/or for treating or preventing hyperinsulinemia and/or insulin resistance;
- preventing, slowing progression of, delaying, or treating new onset diabetes after transplantation (NODAT) and/or post-transplant metabolic syndrome (PTMS);
- preventing, delaying, or reducing NODAT and/or PTMS associated complications including micro- and macrovascular diseases and events, graft rejection, infection, and death;
- treating diabetes associated with cystic fibrosis
- treating hyperuricemia and hyperuricemia associated conditions;
- treating or prevention kidney stones;
- treating hyponatremia;

in a patient in need thereof characterized in that the SGLT2 inhibitor is administered, for example in combination or alternation or sequentially, with a GLP-1 receptor agonist.

According to another aspect of the invention, there is provided the use of a GLP-1 receptor agonist for the manufacture of a medicament for

- treating diabetes mellitus;
- preventing, slowing progression of, delaying or treating of a condition or disorder selected from the group consisting of complications of diabetes mellitus;
- preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity and metabolic syndrome; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance (IGT), impaired fasting blood glucose (IFG), insulin resistance and/or from metabolic syndrome to type 2 diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, arteriosclerosis, myocardial infarction, stroke and peripheral arterial occlusive disease; or
- reducing body weight and/or body fat or preventing an increase in body weight and/or body fat or facilitating a reduction in body weight and/or body fat; or
- preventing, slowing, delaying or treating diseases or conditions attributed to an abnormal accumulation of liver fat; or
- maintaining and/or improving the insulin sensitivity and/or for treating or preventing hyperinsulinemia and/or insulin resistance;

in a patient in need thereof characterized in that the insulin is administered, for example in combination or alternation or sequentially, with an SGLT2 inhibitor.

According to another aspect of the invention, there is provided the use of a pharmaceutical composition according to the present invention for the manufacture of a medicament for a therapeutic and preventive method as described hereinbefore and hereinafter.

## Definitions

The term **"active ingredient"** of a pharmaceutical composition according to the present invention means the SGLT2 inhibitor and/or the long acting insulin according to the present invention.

The term **"body mass index"** or **"BMI"** of a human patient is defined as the weight in kilograms divided by the square of the height in meters, such that BMI has units of $kg/m^2$.

The term **"overweight"** is defined as the condition wherein the individual has a BMI greater than or 25 $kg/m^2$ and less than 30 $kg/m^2$. The terms "overweight" and "pre-obese" are used interchangeably.

The term **"obesity"** is defined as the condition wherein the individual has a BMI equal to or greater than 30 kg/$m^2$. According to a WHO definition the term obesity may be categorized as follows: the term "class I obesity" is the condition wherein the BMI is equal to or greater than 30 kg/$m^2$ but lower than 35 kg/$m^2$; the term "class II obesity" is the condition wherein the BMI is equal to or greater than 35 kg/$m^2$ but lower than 40 kg/$m^2$; the term "class III obesity" is the condition wherein the BMI is equal to or greater than 40 kg/$m^2$.

The term **"visceral obesity"** is defined as the condition wherein a waist-to-hip ratio of greater than or equal to 1.0 in men and 0.8 in women is measured. It defines the risk for insulin resistance and the development of pre-diabetes.

The term **"abdominal obesity"** is usually defined as the condition wherein the waist circumference is > 40 inches or 102 cm in men, and is > 35 inches or 94 cm in women. With regard to a Japanese ethnicity or Japanese patients abdominal obesity may be defined as waist circumference ≥ 85 cm in men and ≥ 90 cm in women (see e.g. investigating committee for the diagnosis of metabolic syndrome in Japan).

The term **"euglycemia"** is defined as the condition in which a subject has a fasting blood glucose concentration within the normal range, greater than 70 mg/dL (3.89 mmol/L) and less than 100 mg/dL (5.6 mmol/L). The word "fasting" has the usual meaning as a medical term.

The term **"hyperglycemia"** is defined as the condition in which a subject has a fasting blood glucose concentration above the normal range, greater than 100 mg/dL (5.6 mmol/L). The word "fasting" has the usual meaning as a medical term.

The term **"hypoglycemia"** is defined as the condition in which a subject has a blood glucose concentration below the normal range, in particular below 70 mg/dL (3.89 mmol/L) or even below 60 mg/dl.

The term **"postprandial hyperglycemia"** is defined as the condition in which a subject has a 2 hour postprandial blood glucose or serum glucose concentration greater than 200 mg/dL (11.1 mmol/L).

The term **"impaired fasting blood glucose"** or **"IFG"** is defined as the condition in which a subject has a fasting blood glucose concentration or fasting serum glucose concentration in a range from 100 to 125 mg/dl (i.e. from 5.6 to 6.9 mmol/l), in particular greater than 110 mg/dL and less than 126 mg/dl (7.00 mmol/L). A subject with "normal fasting glucose" has a fasting glucose concentration smaller than 100 mg/dl, i.e. smaller than 5.6 mmol/l.

The term **"impaired glucose tolerance"** or **"IGT"** is defined as the condition in which a subject has a 2 hour postprandial blood glucose or serum glucose concentration greater than 140 mg/dl (7.8 mmol/L) and less than 200 mg/dL (11.11 mmol/L). The abnormal glucose tolerance, i.e. the 2 hour postprandial blood glucose or serum glucose concentration can be measured as the blood sugar level in mg of glucose per dL of plasma 2 hours after taking 75 g of glucose after a fast. A subject with "normal glucose tolerance" has a 2 hour postprandial blood glucose or serum glucose concentration smaller than 140 mg/dl (7.8 mmol/L).

The term **"hyperinsulinemia"** is defined as the condition in which a subject with insulin resistance, with or without euglycemia, has fasting or postprandial serum or plasma insulin concentration elevated above that of normal, lean individuals without insulin resistance, having a waist-to-hip ratio < 1.0 (for men) or < 0.8 (for women).

The terms "insulin-sensitizing", "insulin resistance-improving" or "insulin resistance-lowering" are synonymous and used interchangeably.

The term "**insulin resistance**" is defined as a state in which circulating insulin levels in excess of the normal response to a glucose load are required to maintain the euglycemic state (Ford ES, et al. JAMA. (2002) 287:356-9). A method of determining insulin resistance is the euglycaemic-hyperinsulinaemic clamp test. The ratio of insulin to glucose is determined within the scope of a combined insulin-glucose infusion technique. There is found to be insulin resistance if the glucose absorption is below the 25th percentile of the background population investigated (WHO definition). Rather less laborious than the clamp test are so called minimal models in which, during an intravenous glucose tolerance test, the insulin and glucose concentrations in the blood are measured at fixed time intervals and from these the insulin resistance is calculated. With this method, it is not possible to distinguish between hepatic and peripheral insulin resistance.

Furthermore, insulin resistance, the response of a patient with insulin resistance to therapy, insulin sensitivity and hyperinsulinemia may be quantified by assessing the "homeostasis model assessment to insulin resistance (HOMA-IR)" score, a reliable indicator of insulin resistance (Katsuki A, et al. Diabetes Care 2001; 24: 362-5). Further reference is made to methods for the determination of the HOMA-index for insulin sensitivity (Matthews et al., Diabetologia 1985, 28: 412-19), of the ratio of intact proinsulin to insulin (Forst et al., Diabetes 2003, 52(Suppl.1): A459) and to an euglycemic clamp study. In addition, plasma adiponectin levels can be monitored as a potential surrogate of insulin sensitivity. The estimate of insulin resistance by the homeostasis assessment model (HOMA)-IR score is calculated with the formula (Galvin P, et al. Diabet Med 1992;9:921-8):

$$\text{HOMA-IR} = [\text{fasting serum insulin } (\mu U/mL)] \times [\text{fasting plasma glucose}(mmol/L)/22.5]$$

As a rule, other parameters are used in everyday clinical practice to assess insulin resistance. Preferably, the patient's triglyceride concentration is used, for example, as increased triglyceride levels correlate significantly with the presence of insulin resistance.

Patients with a predisposition for the development of IGT or IFG or type 2 diabetes are those having euglycemia with hyperinsulinemia and are by definition, insulin resistant. A typical patient with insulin resistance is usually overweight or obese, but this is not always the case. If insulin resistance can be detected, this is a particularly strong indication of the presence of pre-diabetes. Thus, it may be that in order to maintain glucose homoeostasis a person have e.g. 2-3 times as high endogenous insulin production as a healthy person, without this resulting in any clinical symptoms.

The methods to investigate the **function of pancreatic beta-cells** are similar to the above methods with regard to insulin sensitivity, hyperinsulinemia or insulin resistance: An improvement of beta-cell function can be measured for example by determining a HOMA-index for beta-cell function (Matthews et al., Diabetologia 1985, 28: 412-19), the ratio of intact proinsulin to insulin (Forst et al., Diabetes 2003, 52(Suppl.1): A459), the insulin/C-peptide secretion after an oral glucose tolerance test or a meal tolerance test, or by employing a hyperglycemic clamp study and/or minimal modeling after a frequently sampled intravenous glucose tolerance test (Stumvoll et al., Eur J Clin Invest 2001, 31: 380-81).

The term "**pre-diabetes**" is the condition wherein an individual is pre-disposed to the development of type 2 diabetes. Pre-diabetes extends the definition of impaired glucose tolerance to include individuals with a fasting blood glucose within the high normal range ≥ 100 mg/dL (J. B. Meigs, et al. Diabetes 2003; 52:1475-1484) and fasting hyperinsulinemia (elevated plasma insulin concentration). The scientific and medical basis for identifying pre-diabetes as a serious health threat is laid out in a Position Statement entitled "The Prevention or Delay of Type 2 Diabetes" issued jointly by the American Diabetes Association and the National Institute of Diabetes and Digestive and Kidney Diseases (Diabetes Care 2002; 25:742-749).

Individuals likely to have insulin resistance are those who have two or more of the following attributes: 1) overweight or obese, 2) high blood pressure, 3) hyperlipidemia, 4) one or more 1st degree relative with a diagnosis of IGT or IFG or type 2 diabetes. Insulin resistance can be confirmed in these individuals by calculating the HOMA-IR score. For the purpose of this invention, insulin resistance is defined as the clinical condition in which an individual has a HOMA-IR score > 4.0 or a HOMA-IR score above the upper limit of normal as defined for the laboratory performing the glucose and insulin assays.

The term **"type 1 diabetes"** is defined as the condition in which a subject has, in the presence of autoimmunity towards the pancreatic beta-cell or insulin, a fasting blood glucose or serum glucose concentration greater than 125 mg/dL (6.94 mmol/L). If a glucose tolerance test is carried out, the blood sugar level of a diabetic will be in excess of 200 mg of glucose per dL (11.1 mmol/l) of plasma 2 hours after 75 g of glucose have been taken on an empty stomach, in the presence of autoimmunity towards the pancreatic beta cell or insulin. In a glucose tolerance test 75 g of glucose are administered orally to the patient being tested after 10-12 hours of fasting and the blood sugar level is recorded immediately before taking the glucose and 1 and 2 hours after taking it. The presence of autoimmunity towards the pancreatic beta-cell may be observed by detection of circulating islet cell autoantibodies ["type 1A diabetes mellitus"], i.e., at least one of: GAD65 [glutamic acid decarboxylase-65], ICA [islet-cell cytoplasm], IA-2 [intracytoplasmatic domain of the tyrosine phosphatase-like protein IA-2], ZnT8 [zinc-transporter-8] or anti-insulin; or other signs of autoimmunity without the presence of typical circulating autoantibodies [type 1 B diabetes], i.e. as detected through pancreatic biopsy or imaging). Typically a genetic predisposition is present (e.g. HLA, *INS* VNTR and *PTPN22*), but this is not always the case.

The term **"type 2 diabetes"** is defined as the condition in which a subject has a fasting blood glucose or serum glucose concentration greater than 125 mg/dL (6.94 mmol/L). The measurement of blood glucose values is a standard procedure in routine medical analysis. If a glucose tolerance test is carried out, the blood sugar level of a diabetic will be in excess of 200 mg of glucose per dL (11.1 mmol/l) of plasma 2 hours after 75 g of glucose have been taken on an empty stomach. In a glucose tolerance test 75 g of glucose are administered orally to the patient being tested after 10-12 hours of fasting and the blood sugar level is recorded immediately before taking the glucose and 1 and 2 hours after taking it. In a healthy subject, the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg, this is regarded as abnormal glucose tolerance.

The term **"late stage type 2 diabetes mellitus"** includes patients with a secondary drug failure, indication for insulin therapy and progression to micro- and macrovascular complications e.g. diabetic nephropathy, or coronary heart disease (CHD).

The term **"HbA1c"** refers to the product of a non-enzymatic glycation of the haemoglobin B chain. Its determination is well known to one skilled in the art. In monitoring the treatment of diabetes mellitus the HbA1c value is of exceptional importance. As its production depends essentially on the blood sugar level and the life of the erythrocytes, the HbA1c in the sense of a "blood sugar memory" reflects the average blood sugar levels of the preceding 4-6 weeks. Diabetic patients whose HbA1c value is consistently well adjusted by intensive diabetes treatment (i.e. < 6.5 % of the total haemoglobin in the sample), are significantly better protected against diabetic microangiopathy. For example,

metformin on its own achieves an average improvement in the HbA1 c value in the diabetic of the order of 1.0 - 1.5 %. This reduction of the HbA1C value is not sufficient in all diabetics to achieve the desired target range of < 6.5 % and preferably < 6 % HbA1c.

The term **"insufficient glycemic control"** or "inadequate glycemic control" in the scope of the present invention means a condition wherein patients show HbA1c values above 6.5 %, in particular above 7.0 %, even more preferably above 7.5 %, especially above 8 %.

The **"metabolic syndrome"**, also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel I I I) JAMA: Journal of the American Medical Association (2001) 285:2486-2497), diagnosis of the metabolic syndrome is made when three or more of the following risk factors are present:

1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women;
2. Triglycerides: ≥150 mg/dL
3. HDL-cholesterol < 40 mg/dL in men
4. Blood pressure ≥ 130/85 mm Hg (SBP ≥ 130 or DBP ≥ 85)
5. Fasting blood glucose ≥ 100 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/Main, 2000.

According to a commonly used definition, **hypertension** is diagnosed if the systolic blood pressure (SBP) exceeds a value of 140 mm Hg and diastolic blood pressure (DBP) exceeds a value of 90 mm Hg. If a patient is suffering from manifest diabetes it is currently recommended that the systolic blood pressure be reduced to a level below 130 mm Hg and the diastolic blood pressure be lowered to below 80 mm Hg.

The definitions of **NODAT** (new onset diabetes after transplantation) and **PTMS** (post-transplant metabolic syndrome) follow closely that of the American Diabetes Association diagnostic criteria for type 2 diabetes, and that of the International Diabetes Federation (IDF) and the American Heart Association/National Heart, Lung, and Blood Institute, for the metabolic syndrome. NODAT and/or PTMS are associated with an increased risk of micro-and macrovascular disease and events, graft rejection, infection, and death. A number of predictors have been identified as potential risk factors related to NODAT and/or PTMS including a higher age at transplant, male gender, the pre-transplant body mass index, pre-transplant diabetes, and immunosuppression.

The term **"gestational diabetes"** (diabetes of pregnancy) denotes a form of the diabetes which develops during pregnancy and usually ceases again immediately after the birth. Gestational diabetes is diagnosed by a screening test which is carried out between the 24th and 28th weeks of pregnancy. It is usually a simple test in which the blood sugar level is measured one hour after the administration of 50 g of glucose solution. If this 1 h level is above 140 mg/dl, gestational diabetes is suspected. Final confirmation may be obtained by a standard glucose tolerance test, for example with 75 g of glucose.

The term **"hyperuricemia"** denotes a condition of high serum total urate levels. In human blood, uric acid concentrations between 3.6 mg/dL (ca. 214 μmol/L) and 8.3 mg/dL (ca. 494 μmol/L) are considered normal by the American Medical Association. High serum total urate levels, or hyperuricemia, are often associated with several maladies. For example, high serum total urate levels can lead to a type of arthritis in the joints kown as gout. Gout is a condition created by a build up of monosodium urate or uric acid crystals on the articular cartilage of joints, tendons and surrounding tissues due to elevated concentrations of total urate levels in the blood stream. The build up of urate or uric acid on these tissues provokes an inflammatory reaction of these tissues. Saturation levels of uric acid in urine may result in kidney stone formation when the uric acid or urate crystallizes in the kidney. Additionally, high serum total urate levels are often associated with the so-called metabolic syndrome, including cardiovascular disease and hypertension.

The term **"hyponatremia"** denotes a condition of a positive balance of water with or without a deficit of sodium, which is recognized when the plasma sodium falls below the level of 135 mml/L. Hyponatremia is a condition which can occur in isolation in individuals that over-consume water; however, more often hyponatremia is a complication of medication or other underlying medical condition that leas to a diminished excretion of water. Hyponatremia may lead to water intoxication, which occurs when the normal tonicity of extracellular fluid falls below the safe limit, due to retention of excess water. Water intoxication is a potentially fatal disturbance in brain function. Typical symptoms of water intoxication include nausea, vomiting, headache and malaise.

The term **"SGLT2 inhibitor"** in the scope of the present invention relates to a compound, in particular to a glucopyranosyl-derivative, i.e. compound having a glucopyranosyl-moiety, which shows an inhibitory effect on the sodium-glucose transporter 2 (SGLT2), in particular the human SGLT2. The inhibitory effect on hSGLT2 measured as IC50 is prerably below 1000 nM, even more preferably below 100 nM, most preferably below 50 nM. IC50 values of SGLT2 inhibitors are usually above 0.01 nM, or even equal to or above 0.1 nM. The inhibitory effect on hSGLT2 can be determined by methods known in the literature, in particular as described in the application WO 2005/092877 or WO 2007/093610 (pages 23/24), which are incorporated herein by reference in its entirety. The term "SGLT2 inhibitor" also comprises any pharmaceutically acceptable salts thereof, hydrates and solvates thereof, including the respective crystalline forms.

The term **"insulin"** in the scope of the present invention relates to insulin and insulin analogs being used in the therapy of patients, in particular humans, which includes normal insulin, human insulin, insulin derivatives, zinc insulins and insulin analogues, including formulations thereof with modified release profiles. in particular as used in the therapy of humans. The term "insulin" in the scope of the present invention covers the following types of insulins:

- rapid-acting insulins,
- short-acting insulins,
- intermediate-acting insulins,
- long-acting insulins,

and mixtures thereof, for example mixtures of short- or rapid-acting insulins with long-acting insulins. The term "insulin" in the scope of the present invention covers insulins which are administered to the patient via injection, via infusion, including pumps, via inhalation, via oral, via transdermal or other routes of administration.

The term **"GLP-1 receptor agonist"** in the scope of the present invention includes, without being limited, exogenous GLP-1 (natural or synthetic), GLP-1 analogues and other substances (whether peptidic or non-peptidic, e.g. small molecules) which promote signalling through the GLP-1 receptor. The exogenous GLP-1 includes natural and synthetic GLP-1, in particular human GLP-1. The GLP-1 analogues include longer acting analogues also which are resistant to or have reduced susceptibility to enzymatic degradation, for example by DPP-4 and/or NEP 24.11.

The terms **"treatment"** and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

The terms **"prophylactically treating"**, "preventivally treating" and "preventing" are used interchangeably and comprise a treatment of patients at risk to develop a condition mentioned hereinbefore, thus reducing said risk.

**Brief Description of the Figures**

The Figure 1 shows the glucose excursion in ZDF rats after administration of a SGLT2 inhibitor and a GLP-1 receptor agonist.

**Detailed Description**

The aspects according to the present invention, in particular the pharmaceutical compositions, methods and uses, refer to SGLT2 inhibitors and GLP-1 receptor agonists.

The SGLT2 inhibitor is

| | |
|---|---|
| (I.9) | 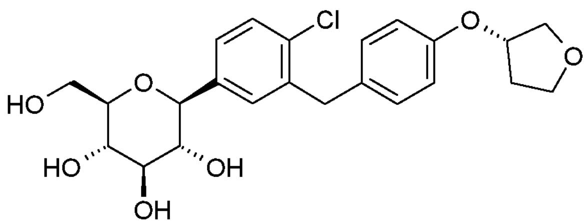 1-chloro-4-(β-D-glucopyranos-1-yl)-2-[4-((*S*)-tetrahydrofuran-3-yloxy)-benzyl]-benzene, |

The aspects according to the present invention, in particular the pharmaceutical compositions, methods and uses, refer to a GLP-1 receptor agonist, which includes exogenous GLP-1 (natural or synthetic), GLP-1 analogues and other substances (whether peptidic or non-peptidic, e.g. small molecules) which promote signalling through the GLP-1 receptor.

Examples of GLP-1 analogues are selected from the group G2 consisting of exenatide (exendin-4); exenatide LAR (long acting release formulation of exenatide); liraglutide; taspoglutide; semaglutide; albiglutide; lixisenatide; dulaglutide; and the PEGylated GLP-1 compound comprising the amino acid sequence according to the claim 1 of WO 2006/124529 (the disclosure of which is incorporated herein) and the GLP-1 derivative comprising the amino acid sequence according to SEQ ID NO:21 as disclosed in the WO 2009/020802 (the disclosure of which is incorporated herein).

Preferred examples of GLP-1 receptor agonists (GLP-1 analogues) of this invention are exenatide, exenatide LAR, liraglutide, taspoglutide, semaglutide, albiglutide, lixisenatide and dulaglutide, in particular exenatide (exendin-4).

GLP-1 analogues have typically significant sequence identity to GLP-1 (e.g. greater than 50%, 75%, 90% or 95%) and may be derivatised, e.g. by conjunction to other proteins (e.g. albumin or IgG-Fc fusion protein) or through chemical modification.

Unless otherwise noted, according to this invention it is to be understood that the definitions of the active agents (including the SGLT2 inhibitors and GLP-1 receptor agonists) mentioned hereinbefore and hereinafter may also contemplate their pharmaceutically acceptable salts, and prodrugs, hydrates, solvates and polymorphic forms thereof. Particularly the terms of the therapeutic agents given herein refer to the respective active drugs. With respect to salts, hydrates and polymorphic forms thereof, particular reference is made to those which are referred to herein.

In an embodiment the combinations, compositions, methods and uses according to this invention relate to combinations wherein the SGLT2 inhibitor and the GLP-1 receptor agonist are preferably selected according to the entries in the Table 1:

Table 1

| compound (I.9) | selected from group G2 |
|---|---|
| compound (I.9) | exenatide |
| compound (I.9) | exenatide LAR |
| compound (I.9) | liraglutide |
| compound (I.9) | taspoglutide |
| compound (I.9) | semaglutide |
| compound (I.9) | albiglutide |
| compound (I.9) | lixisenatide |
| compound (I.9) | dulaglutide |

In a particular embodiment (embodiment E) the combinations, compositions, methods and uses according to this invention relate to combinations wherein the SGLT2 inhibitor is the compound of the formula (I.9).

The combination of an SGLT2 inhibitor and a GLP-1 receptor agonist according to this invention significantly improves the glycemic control, in particular in patients as described hereinafter, compared with a monotherapy using either a SGLT2 inhibitor or a GLP-1 receptor agonist alone. Furthermore the combination of an SGLT2 inhibitor and a GLP-1 receptor agonist according to this invention may allow a reduction of the dose of the GLP-1 receptor agonist compared with a monotherapy of said GLP-1 receptor agonist. With a reduction of the dose of the GLP-1 receptor agonist any side effects associated with the therapy using said GLP-1 receptor agonist may be prevented or attenuated. A dose reduction is beneficial for patients which otherwise would potentially suffer from side effects in a therapy using a higher dose of one or more of the active ingredients, in particular with regard to side effect caused by the GLP-1 receptor agonist. Therefore, the pharmaceutical composition as well as the methods according to the present invention, may show less side effects, thereby making the therapy more tolerable and improving the patients compliance with the treatment. In addition the efficacy of the GLP-1 receptor agonist may be prolonged by a combined treatment with a SGLT2 inhibitor. Therefore the time interval between two applications, for example subcutaneous injections or infusions via a pump, of the GLP-1 receptor agonist may be prolonged. For example in a combination therapy employing a GLP-1 receptor agonist and a SGLT2 inhibitor according to the invention the dose of the GLP-1 receptor agonist, the dose of the SGLT2 inhibitor, the time intervall between two applications of the GLP-1 receptor agonist and the time intervall between the application of the GLP-1 receptor agonist and the SGLT2 inhibitor are chosen such that a good glycemic control is provided to the patient for a given time period, in particular for 24 hours.

When this invention refers to patients requiring treatment or prevention, it relates primarily to treatment and

prevention in humans, but the pharmaceutical composition may also be used accordingly in veterinary medicine in mammals. In the scope of this invention the term "patient" covers adult humans (age of 18 years or older), adolescent humans (age 10 to 17 years) and children (age 6-9 years).

As described hereinbefore by the administration of the pharmaceutical composition according to this invention and in particular in view of the effect of the GLP1-1 receptor agonist and of the SGLT2 inhibitors therein, no gain in weight or even a reduction in body weight may result. Therefore, a treatment or prophylaxis according to this invention is advantageously suitable in those patients in need of such treatment or prophylaxis who are diagnosed of one or more of the conditions selected from the group consisting of overweight and obesity, in particular class I obesity, class II obesity, class III obesity, visceral obesity and abdominal obesity. In addition a treatment or prophylaxis according to this invention is advantageously suitable in those patients in which a weight increase is contraindicated. Any weight increasing effect in the therapy, for example due to the administration of the third antidiabetic agent, may be attenuated or even avoided thereby.

According to an embodiment of the present invention, there is provided a method for improving glycemic control and/or for reducing of fasting plasma glucose, of postprandial plasma glucose and/or of glycosylated hemoglobin HbA1 c in a patient in need thereof who is diagnosed with impaired glucose tolerance (IGT), impaired fasting blood glucose (IFG) with insulin resistance, with metabolic syndrome and/or with type 1 diabetes mellitus or type 2 diabetes mellitus characterized in that a GLP-1 receptor agonist and an SGLT2 inhibitor as defined hereinbefore and hereinafter are administered, for example in combination or alternation or sequentially, to the patient.

According to another embodiment of the present invention, there is provided a method for improving gycemic control in patients, in particular in adult patients, with type 2 diabetes mellitus as an adjunct to diet and exercise characterized by an adiministration of a GLP-1 receptor agonist and an SGLT2 inhibitor as defined hereinbefore and hereinafter, for example in combination or alternation or sequentially, to the patient..

It can be found that by using a pharmaceutical composition according to this invention, an improvement of the glycemic control can be achieved even in those patients who have insufficient glycemic control in particular despite treatment with a GLP-1 receptor agonist, for example despite maximal recommended or tolerated dose of monotherapy with the GLP-1 receptor agonist.

Furthermore, the pharmaceutical composition, the methods and uses according to this invention are particularly suitable in the treatment of patients who are diagnosed having one or more of the following conditions

(a) obesity (including class I, II and/or III obesity), visceral obesity and/or abdominal obesity,
(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressures 85 mm Hg,
(e) a fasting blood glucose level ≥ 100 mg/dL.

It is assumed that patients diagnosed with impaired glucose tolerance (IGT), impaired fasting blood glucose (IFG), with insulin resistance and/or with metabolic syndrome suffer from an increased risk of developing a cardiovascular disease, such as for example myocardial infarction, coronary heart disease, heart insufficiency, thromboembolic events. A glycemic control according to this invention may result in a reduction of the cardiovascular risks.

Furthermore, the pharmaceutical composition, the methods and uses according to this invention are particularly suitable in the treatment of patients after organ transplantation, in particular those patients who are diagnosed having one or more of the following conditions

(a) a higher age, in particular above 50 years,
(b) male gender;
(c) overweight, obesity (including class I, II and/or III obesity), visceral obesity and/or abdominal obesity,
(d) pre-transplant diabetes,
(e) immunosuppression therapy.

Furthermore, the pharmaceutical composition, the methods and the uses according to this invention are particularly suitable in the treatment of patients who are diagnosed having one or more of the following conditions:

(a) hyponatremia, in particular chronical hyponatremia;
(b) water intoxication;
(c) water retention;
(d) plasma sodium concentration below 135 mmol/L.

Furthermore, the pharmaceutical composition, the methods and uses according to this invention are particularly

suitable in the treatment of patients who are diagnosed having one or more of the following conditions:

(a) high serum uric acid levels, in particular greater than 6.0 mg/dL (357 μmol/L);
(b) a history of gouty arthritis, in particular recurrent gouty arthritis;
(c) kidney stones, in particular recurrent kidney stones;
(d) a high propensity for kidney stone formation.

A pharmaceutical composition according to this invention, in particular due to the SGLT2 inhibitor exhibits a good safety profile. Therefore, a treatment according to this invention is advantageous in those patients for which a reduction of the dose of the GLP-1 receptor agonist is recommended.
A pharmaceutical composition according to this invention is particularly suitable in the long term treatment or prophylaxis of the diseases and/or conditions as described hereinbefore and hereinafter, in particular in the long term glycemic control in patients with type 2 diabetes mellitus.
The term "long term" as used hereinbefore and hereinafter indicates a treatment of or administration in a patient within a period of time longer than 12 weeks, preferably longer than 25 weeks, even more preferably longer than 1 year.
Therefore, a particularly preferred embodiment of the present invention provides a method for therapy, preferably oral therapy, for improvement, especially long term improvement, of glycemic control in patients with type 2 diabetes mellitus, especially in patients with late stage type 2 diabetes mellitus, in particular in patients additionally diagnosed of overweight, obesity (including class I, class II and/or class III obesity), visceral obesity and/or abdominal obesity.
Unless otherwise noted, the combination therapy according to the invention may refer to first line, second line or third line therapy, or initial or add-on combination therapy or replacement therapy.
According to one embodiment the the GLP-1 receptor agonist and SGLT2 inhibitor are administered in combination, i.e. simultaneously, for example in one single formulation or in two separate formulations or dosage forms, or in alternation or sequentially, for example successively in two separate formulations or dosage forms. Hence, the administration of one combination partner, i.e. the SGLT2 inhibitor or the GLP-1 receptor agonist, may be prior to, concurrent to, or subsequent to the administration of the other combination partner. In one embodiment, for the combination therapy according to this invention the GLP-1 receptor agonist and the SGLT2 inhibitor are administered in different formulations or different dosage forms. In another embodiment, for the combination therapy according to this invention the SGLT2 inhibitor and the GLP-1 receptor agonist are administered in the same formulation or in the same dosage form.
Therefore according to an embodiment of the present invention there is provided a pharmaceutical composition or fixed dose combination comprising

a) a SGLT2 inhibitor as defined herein, and
b) a GLP-1 receptor agonist as defined herein,

and, optionally, one or more pharmaceutically acceptable carriers and/or diluents.
Within the scope of the present invention, the SGLT2 inhibitor is preferably administered orally or by injection, preferably orally. The GLP-1 receptor agonist is preferably administered by injection, preferably subcutaneously, or by infusion. Other forms of administration are possible and described hereinafter.
Therefore according to another embodiment the present invention provides a pharmaceutical composition, delivery system or device for systemic use, in particular for administration by injection or infusion, for example subcutaneous injection, comprising

a) a SGLT2 inhibitor as defined herein, and, optionally,
b) a GLP-1 receptor agonist as defined herein,

and, optionally, one or more pharmaceutically acceptable carriers and/or diluents.
It will be appreciated that the amount of the SGLT2 inhibitor and the GLP-1 receptor agonist according to this invention to be administered to the patient and required for use in treatment or prophylaxis according to the present invention will vary with the route of administration, the nature and severity of the condition for which treatment or prophylaxis is required, the age, weight and condition of the patient, concomitant medication and will be ultimately at the discretion of the attendant physician. In general, however, the SGLT2 inhibitor, and the GLP-1 receptor agonist according to this invention are included in the pharmaceutical composition or dosage form in an amount sufficient that by their administration in combination and/or alternation or sequentially the glycemic control in the patient to be treated is improved.
For the treatment of hyperuricemia or hyperuricemia associated conditions the SGLT2 inhibitor according to this invention is included in the pharmaceutical composition or dosage form in an amount sufficient that is sufficient to treat hyperuricemia without disturbing the patient’s plasma glucose homeostasis, in particular without inducing hypoglycemia.

For the treatment or prevention of kidney stones the SGLT2 inhibitor according to this invention is included in the pharmaceutical composition or dosage form in an amount sufficient that is sufficient to treat or prevent kidney stones without disturbing the patient's plasma glucose homeostasis, in particular without inducing hypoglycemia.

For the treatment of hyponatremia and associated conditions the SGLT2 inhibitor according to this invention is included in the pharmaceutical composition or dosage form in an amount sufficient that is sufficient to treat hyponatremia or the associated conditions without disturbing the patient's plasma glucose homeostasis, in particular without inducing hypoglycemia.

In the following preferred range ≥ of the amount of the SGLT2 inhibitor, and the GLP-1 receptor agonist to be employed in the pharmaceutical composition and the methods and uses according to this invention are described. These range ≥ refer to the amounts to be administered per day with respect to an adult patient, in particular to a human being, for example of approximately 70 kg body weight, and can be adapted accordingly with regard to an administration 1 or 2 times daily and with regard to other routes of administration and with regard to the age of the patient. The range ≥ of the dosage and amounts are calculated for the inidividual active moiety. Advantageously, the combination therapy according to the present invention utilizes lower dosages of the individual SGLT2 inhibitor, and of the individual GLP-1 receptor agonist used in monotherapy or used in conventional therapeutics, thus avoiding possible adverse side effects incurred when those agents are used as monotherapies.

In general, the amount of the SGLT2 inhibitor in the pharmaceutical composition, methods and uses according to this invention is preferably in the range from 1/5 to 1/1 of the amount usually recommended for a monotherapy using said SGLT2 inhibitor.

The preferred dosage range of the SGLT2 inhibitor is in the range from 0.5 mg to 200 mg, even more preferably from 1 to 100 mg, most preferably from 1 to 50 mg per day. The oral administration is preferred. Therefore, a pharmaceutical composition may comprise the hereinbefore mentioned amounts, in particular from 1 to 50 mg or 1 to 25 mg. Particular dosage strengths (e.g. per tablet or capsule) are for example 1,2.5, 5, 7.5, 10, 12.5, 15, 20, 25 or 50 mg of the compound of the formula (I), in particular of the compound (I.9). The application of the active ingredient may occur one, two or three times a day, preferably once a day.

In general, the amount of the GLP-1 receptor agonist in the pharmaceutical composition, methods and uses according to this invention is preferably in the range from 1/5 to 1/1 of the amount usually recommended for a monotherapy using said GLP-1 receptor agonist.

The GLP-1 receptor agonist is typically administered by subcutaneous injection, e.g. ranging from once, twice, three or more times daily. For example the GLP-1 receptor agonist is administered subcutaneously via injection about 30 to 60 minutes before a meal. Suitable doses and dosage forms of the GLP-1 receptor agonist may be determined by a person skilled in the art.

Exenatide is usually administered once, twice or more times, preferably twice daily by subcutaneous injection with a dose in the range form 5 to 30 μg, particularly 5 to 20μg, preferably 5 to 10 μg. Specific dosage strengths are for example 5 or 10 μg per administration. A tradename of exenatide is Byetta. For example exenatide is administered twice daily via subcutaneous injection each of 5 μg. The therapy is continued for at least one month. The dose may be increased to 10 μg twice daily. The time of each injection is preferably within 60 minutes before a meal, for example before a meal in the morning and before a meal in the evening.

Exenatide LAR is usually administered once weekly by subcutaneous injection (0.1-3 mg, particularly 0.5 mg to 2.0 mg, specific dosage strengths are 0.8 mg or 2.0 mg).

Liraglutide is usually administered once daily by subcutaneous injection (0.5-3 mg, particularly 0.5 mg to 2 mg, specific dosage strengths are 0.6 mg, 0.9 mg, 1.2 mg or 1.8 mg).

Taspoglutide is usually administered once weekly by subcutaneous injection (1-30 mg, specific dosage strengths are 1 mg, 8 mg, 10 mg, 20 mg or 30 mg).

Semaglutide is usually administered once weekly by subcutaneous injection (0.1-1.6 mg). Albiglutide is administered once weekly by subcutaneous injection (4-30 mg, specific dosage strengths are 4 mg, 15 mg or 30 mg).

Lixisenatide is usually administered once daily by subcutaneous injection (10-20 μg, specific dosage strengths are 10 μg, 15 μg or 20 μg).

Dulaglutide is usually administered once weekly by subcutaneous injection (0.25-3 mg, specific dosage strengths are 0.25 mg, 0.5 mg, 0.75 mg, 1.0 mg, 1.5 mg, 2.0 mg or 3.0 mg).

In the methods and uses according to the present invention the GLP-1 receptor agonist and the SGLT2 inhibitor are administered in combination or alternation or sequentially. The term "administration in combination" means that the active ingredients are administered at the same time, i.e. simultaneously, or essentially at the same time. The term "administration in alternation" means that at first one of the two active ingredients, i.e. the SGLT2 inhibitor or the GLP-1 receptor agonist, is administered and after a period of time the other active ingredient, i.e. the GLP-1 receptor agonist or the SGLT2 inhibitor, is administered whereby this administration scheme may be repeated one or more times. The period of time between the administration of the first and of the second active ingredient may be in the range from 1 min

to 12 hours. The administration which is in combination or in alternation may be once, twice, three times or four times daily, preferably once or twice daily. The term "sequentially" means that to a patient the first active ingredient, in particular the GLP-1 receptor agonist, is administered to the patient one or more times in a first period of time followed by an administration of the second active ingredient, in particular the SGLT2 inhibitor which is adminstered to the patient one or more times in a second period of time. In other words the term "sequentially" includes a first therapy, in particular with the GLP-1 receptor agonist, in a first period of time followed by a second therapy, in particular with the SGLT2 inhibitor, in a second period of time. The time periods may be in a range from one to more days, one to more weeks or one to more months. According to one embodiment the first and the second period of time is in a range from one to more weeks, in particular one to more months.
A pharmaceutical composition which is present as a separate or multiple dosage form, preferably as a kit of parts, is useful in combination therapy to flexibly suit the individual therapeutic needs of the patient.

According to a first embodiment a preferred kit of parts comprises

(a) a first containment containing a dosage form comprising the SGLT2 inhibitor and at least one pharmaceutically acceptable carrier, and
(b) a second containment containing a dosage form comprising the GLP-1 receptor agonist and at least one pharmaceutically acceptable carrier.

A further aspect of the present invention is a manufacture comprising the pharmaceutical composition being present as separate dosage forms according to the present invention and a label or package insert comprising instructions that the separate dosage forms are to be administered in combination or alternation or sequentially.

According to a first embodiment a manufacture comprises (a) a pharmaceutical composition comprising a SGLT2 inhibitor according to the present invention and (b) a label or package insert which comprises instructions that the medicament may or is to be administered, for example in combination or alternation or sequentially, with a medicament comprising a GLP-1 receptor agonist according to the present invention.

According to a second embodiment a manufacture comprises (a) a pharmaceutical composition comprising a GLP-1 receptor agonist according to the present invention and (b) a label or package insert which comprises instructions that the medicament may or is to be administered, for example in combination or alternation or sequentially, with a medicament comprising a SGLT2 inhibitor according to the present invention or with a medicament comprising both a SGLT2 inhibitor.

The desired dose of the pharmaceutical composition according to this invention may conveniently be presented in a once daily or as divided dose administered at appropriate intervals, for example as two, three or more doses per day.

The pharmaceutical composition may be formulated for oral, parenteral (including subcutaneous) or other routes of administration in liquid or solid form. Oral administration of the SGLT2 inhibitor is preferred. The formulations may, where appropriate, be conveniently presented in discrete dosage units and may be prepared by any of the methods well known in the art of pharmacy. All methods include the step of bringing into association the active ingredient with one or more pharmaceutically acceptable carriers, like liquid carriers or finely divided solid carriers or both, and then, if necessary, shaping the product into the desired formulation. Examples of pharmaceutical compositions comprising the SGLT2 inhibitor compound (1.9) are described in WO 2010/092126. Examples of pharmaceutical compositions comprising the SGLT2 inhibitor compound (1.9) and linagliptin are described in WO 2010/092124.

Injectable formulations of the GLP-1 receptor agonists of this invention may be prepared according to known formulation techniques, e.g. using suitable liquid carriers, which usually comprise sterile water, and, optionally, further additives e.g. for aiding solubility or for preservation or the like, to obtain injectable solutions or suspensions.

The pharmaceutical composition may be formulated in the form of solutions, suspensions, emulsions, tablets, granules, fine granules, powders, capsules, caplets, soft capsules, pills, oral solutions, syrups, dry syrups, chewable tablets, troches, effervescent tablets, drops, fast dissolving tablets, oral fast-dispersing tablets, etc.. Preferably the pharmaceutical composition of the SGLT2 inhibitor is in the form of tablets.

The pharmaceutical composition and the dosage forms preferably comprises one or more pharmaceutical acceptable carriers. Preferred carriers must be "acceptable" in the sense of being compatible with the other ingredients of the formulation and not deleterious to the recipient thereof. Examples of pharmaceutically acceptable carriers are known to the one skilled in the art.

The pharmaceutical composition according to the invention may also be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredients may be in powder form, obtained by aseptic isolation of sterile solid or by lyophilisation from solution, for constitution with a suitable vehicle, e.g. sterile, pyrogen-free water, before use.

Injectable formulations of the GLP-1 receptor agonist and/or the SGLT2 inhibitor of this invention (particularly for subcutaneous use) may be prepared according to known formulation techniques, e.g. using suitable liquid carriers, which usually comprise sterile water, and, optionally, further additives such as e.g. preservatives, pH adjusting agents, buffering agents, isotoning agents, solubility aids and/or tensides or the like, to obtain injectable solutions or suspensions. In addition, injectable formulations may comprise further additives, for example salts, solubility modifying agents or precipitating agents which retard release of the drug(s). In further addition, injectable GLP-1 receptor agonist formulations may comprise stabilizing agents. The component GLP-1 receptor agonist of the combination according to the invention is preferably administered by injection (preferably subcutaneously).

For further details on dosage forms, formulations and administration of SGLT2 inhibitors of this invention and/or GLP-1 receptor agonist of this invention, reference is made to scientific literature and/or published patent documents, particularly to those cited herein.

The pharmaceutical compositions (or formulations) may be packaged in a variety of ways. Generally, an article for distribution includes one or more containers that contain the one or more pharmaceutical compositions in an appropriate form. Tablets are typically packed in an appropriate primary package for easy handling, distribution and storage and for assurance of proper stability of the composition at prolonged contact with the environment during storage. Primary containers for tablets may be bottles or blister packs.

Solutions for injection may be available in typical suitable presentation forms such as vials, cartridges or prefilled (disposable) pens, which may be further packaged.

The article may further comprise a label or package insert, which refers to instructions customarily included in commercial packages of therapeutic products, that may contain information about the indications, usage, dosage, administration, contraindications and/or warnings concerning the use of such therapeutic products. In one embodiment, the label or package inserts indicates that the composition can be used for any of the purposes described hereinbefore or hereinafter.

The pharmaceutical compositions and methods according to this invention show advantageous effects in the treatment and prevention of those diseases and conditions as described hereinbefore compared with pharmaceutical compositions and methods which comprise only one of the two active ingredients. Additional advantageous effects may be seen for example with respect to efficacy, dosage strength, dosage frequency, pharmacodynamic properties, pharmacokinetic properties, fewer adverse effects, convenience, compliance, etc..

Methods for the manufacture of SGLT2 inhibitors according to this invention and of prodrugs thereof are known to the one skilled in the art. Advantageously, the compounds according to this invention can be prepared using synthetic methods as described in the literature, including patent applications as cited hereinbefore. Preferred methods of manufacture are described in the WO 2006/120208 and WO 2007/031548. With regard to the preferred compound (1.9) an advantageous crystalline form is described in the international patent application WO 2006/117359 which hereby is incorporated herein in its entirety.

With respect to GLP-1 receptor agonists the methods of synthesis are known to the skilled person and as described in the scientific literature and/ or in published patent documents, particularly in those cited hereinbefore.

The active ingredients, in particular the GLP-1 receptor agonist, may be present in the form of a pharmaceutically acceptable salt. The active ingredients or a pharmaceutically acceptable salt thereof may be present in the form of a solvate such as a hydrate or alcohol adduct.

Any of the above mentioned combinations and methods within the scope of the invention may be tested by animal models known in the art. In the following, *in vivo* experiments are described which are suitable to evaluate pharmacologically relevant properties of pharmaceutical compositions and methods according to this invention:

> Pharmaceutical compositions and methods according to this invention can be tested in genetically hyperinsulinemic or diabetic animals like db/db mice, ob/ob mice, Zucker Fatty (fa/fa) rats or Zucker Diabetic Fatty (ZDF) rats. In addition, they can be tested in animals with experimentally induced diabetes like HanWistar or Sprague Dawley rats pretreated with streptozotocin.

The effect on glycemic control of the combinations according to this invention can be tested after single dosing of the SGLT2 inhibitor and the GLP-1 receptor agonist alone and in combination in an oral glucose tolerance test in the animal models described hereinbefore. The time course of blood glucose is followed after an oral glucose challenge in overnight fasted animals. The combinations according to the present invention improve glucose excursion compared to each monotherapy as measured by reduction of peak glucose concentrations or reduction of glucose AUC. In addition, after multiple dosing of the SGLT2 inhibitor and the GLP-1 receptor agonist alone and in combination in the animal models described hereinbefore, the effect on glycemic control can be determined by measuring the HbA1c value in blood. The combinations according to this invention may reduce HbA1c compared to each monotherapy.

The possible dose reduction of one or both of the SGLT2 inhibitor and the GLP-1 receptor agonist can be tested by the effect on glycemic control of lower doses of the combinations and monotherapies in the animal models described

hereinbefore. The combinations according to this invention at the lower doses may improve glycemic control compared to placebo treatment whereas the monotherapies at lower doses do not.

A superior effect of the combination of a SGLT2 inhibitor and a GLP-1 receptor agonist according to the present invention on beta-cell regeneration and neogenesis can be determined after multiple dosing in the animal models described hereinbefore by measuring the increase in pancreatic insulin content, or by measuring increased beta-cell mass by morphometric analysis after immunohistochemical staining of pancreatic sections, or by measuring increased glucose-stimulated insulin secretion in isolated pancreatic islets.

## Pharmacological Examples

The following example shows the beneficial effect on glycemic control of the combination according to the present invention.

### Example 1:

The following example shows the beneficial effect on glycemic control of the combination of a glucopyranosyl-substituted benzene derivative and the GLP-1 receptor agonist exendin-4 (1-39) as compared to the respective monotherapies. All experimental protocols concerning the use of laboratory animals were reviewed by a federal Ethics Committee and approved by governmental authorities. An oral glucose tolerance test was performed in overnight fasted 12-weeks old male Zucker diabetic fatty (ZDF) rats (ZDF/CRL-Lepr$^{fa}$). A pre-dose blood sample was obtained by tail bleed. Blood glucose was measured with a glucometer, and the animals were randomized for blood glucose (n = 5 / group). Subsequently, the groups received a single oral administration of either vehicle alone (0.5% aqueous hydroxyethylcellulose) or this vehicle containing the glucopyranosyl-substituted benzene derivative. Fifteen minutes later, the groups received a single subcutaneous injection of either vehicle alone (physiological saline solution) or this vehicle containing exendin-4. The animals were orally dosed with glucose (2 g/kg) 30 min after the subcutaneous injection. Blood glucose was measured in tail blood 15 min, 30 min, 60 min, 90 min, and 120 min thereafter. Glucose excursion was quantified by calculating the reactive glucose AUC. The data are presented as mean ± S.E.M. The two-sided unpaired Student's t-test was used for statistical comparison of the groups. A p value < 0.05 was considered to show a statistically significant difference. The result is shown in the Figure 1. Therein "Cpd. A" denotes the glucopyranosyl-substituted benzene derivative (compound (1.9)) at a dose of 3 mg/kg. Exendin-4 was dosed at 0.01 mg/kg. The term "Combination A + Exendin-4" denotes the combination of the glucopyranosyl-substituted benzene derivative and exendin-4 at the same doses. P values versus control are indicated by symbols above the bars. P values of the combination versus the monotherapies are indicated below the figure (*, p < 0.05; **, p < 0.01; ***, p < 0.001). The glucopyranosyl-substituted benzene derivative reduced glucose excursion by 57%, and exendin-4 reduced glucose excursion by 40%. The combination decreased glucose excursion in the oral glucose tolerance test by 83%, and this reduction in glucose AUC was statistically significant versus each monotherapy.

## Claims

1. A pharmaceutical composition comprising

   (a) an SGLT2 inhibitor, and
   (b) a GLP-1 receptor agonist,

   without a third antidiabetic agent,
   wherein the SGLT2 inhibitor is 1-chloro-4-(β-D-glucopyranos-1-yl)-2-[4-(*(S)*-tetrahydrofuran-3-yloxy)-benzyl]-benzene and wherein the amount of the SGLT2 inhibitor is from 1 to 25 mg.

2. The pharmaceutical composition according to claim 1 wherein the GLP-1 receptor agonist is selected from the group consisting of exenatide, exenatide LAR, liraglutide, taspoglutide, semaglutide, albiglutide, lixisenatide and dulaglutide:

3. The pharmaceutical composition according to the claim 2 wherein the GLP-1 receptor agonist is exenatide or exenatide LAR.

4. The pharmaceutical composition according to one of the previous claims **characterized in that** the composition is suitable for combined or simultaneous or sequential use of the SGLT2 inhibitor and the GLP-1 receptor agonist.

**5.** The pharmaceutical composition according to one of the claims 1 to 4 for use in a method for treating a disease or condition selected from the group consisting of diabetes mellitus, type 2 diabetes mellitus and a disease or condition which requires treatment with insulin or a GLP-1 receptor agonist in a patient.

**6.** The pharmaceutical composition according to one of the claims 1 to 4 for use in a method for treating a disease or condition selected from the group consisting of

- for treating diabetes mellitus;
- for treating diabetes mellitus, where treatment with insulin or a GLP-1 receptor agonist is required;
- for treating, preventing or reducing the risk of hypoglycemia;
- for preventing, slowing progression of, delaying or treating of a condition or disorder selected from the group consisting of complications of diabetes mellitus;
- preventing, slowing the progression of, delaying or treating a metabolic disorder selected from the group consisting of type 1 diabetes mellitus, type 2 diabetes mellitus, impaired glucose tolerance, impaired fasting blood glucose, hyperglycemia, postprandial hyperglycemia, overweight, obesity, metabolic syndrome, gestational diabetes, diabetes related to cystic fibrosis; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance, insulin resistance and/or from metabolic syndrome to type 2 diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, diabetic foot, arteriosclerosis, myocardial infarction, accute coronary syndrome, unstable angina pectoris, stable angina pectoris, stroke, peripheral arterial occlusive disease, cardiomyopathy, heart failure, heart rhythm disorders and vascular restenosis; or
- reducing body weight and/or body fat or preventing an increase in body weight and/or body fat or facilitating a reduction in body weight and/or body fat; or
- preventing, slowing, delaying or treating the degeneration of pancreatic beta cells and/or the decline of the functionality of pancreatic beta cells and/or for improving and/or restoring the functionality of pancreatic beta cells and/or restoring the functionality of pancreatic insulin secretion; or
- for preventing, slowing, delaying or treating diseases or conditions attributed to an abnormal accumulation of ectopic fat; or
- maintaining and/or improving the insulin sensitivity and/or for treating or preventing hyperinsulinemia and/or insulin resistance;
- preventing, slowing progression of, delaying, or treating new onset diabetes after transplantation (NODAT) and/or post-transplant metabolic syndrome (PTMS);
- preventing, delaying, or reducing NODAT and/or PTMS associated complications including micro- and macrovascular diseases and events, graft rejection, infection, and death;
- treating hyperuricemia and hyperuricemia associated conditions;
- treating or preventing kidney stones;
- treating hyponatremia.

**7.** The pharmaceutical composition according to one of the claims 1 to 4 for use in a therapeutic method for weight reduction, for reduction of body fat, for preventing an increase of body weight or for attenuating an increase of body weight comprising an administration of the GLP-1 receptor agonist in a patient in need thereof followed by an administration of the SGLT2 inhibitor in said patient.

**8.** The pharmaceutical composition according to one of the claims 1 to 4 for use in a therapeutic method for body weight reduction, for reduction of body fat, for preventing an increase of body weight or for attenuating an increase of body weight wherein the administration of the SGLT2 inhibitor follows a termination or discontinuation of a treatment with the GLP-1 receptor agonist.

**9.** The pharmaceutical composition according to one of the claims 1 to 4 for use in a method for treating a metabolic disorder, in particular of diabetes mellitus and/or complications associated with diabetes mellitus, in a patient who is diagnosed of being overweight or obese, **characterized by** a first therapy comprising the administration of the GLP-1 receptor agonist to the patient followed by a second therapy comprising the administration of the SGLT2 inhibitor to the patient

**10.** The SGLT2 inhibitor 1-chloro-4-(β-D-glucopyranos-1-yl)-2-[4-(*(S)*)-tetrahydrofuran-3-yloxy)-benzyl]-benzene for use in a therapeutic method for body weight reduction, for reduction of body fat, for preventing an increase of body weight or for attenuating an increase of body weight wherein the administration of the SGLT2 inhibitor follows a termination or discontinuation of a treatment with a GLP-1 receptor agonist and wherein the SGLT2 inhbibitor is applied once a day in an amount from 1 to 25 mg.

**11.** The SGLT2 inhibitor 1-chloro-4-(β-D-glucopyranos-1-yl)-2-[4-(*(S)*)-tetrahydrofuran-3-yloxy)-benzyl]-benzene for use in a method for treating a metabolic disorder, in particular of diabetes mellitus and/or complications associated with diabetes mellitus, in a patient who is diagnosed of being overweight or obese, **characterized by** a first therapy comprising the administration of a GLP-1 receptor agonist to the patient followed by a second therapy comprising the administration of the SGLT2 inhibitor to the patient and wherein the SGLT2 inhbibitor is applied once a day in an amount from 1 to 25 mg.

## Patentansprüche

**1.** Pharmazeutische Zusammensetzung, umfassend

(a) einen SGLT2-Inhibitor und
(b) einen GLP-1-Rezeptoragonisten,

ohne ein drittes Antidiabetesmittel,
wobei der SGLT2-Inhibitor 1-Chlor-4-(β-D-glucopyranos-1-yl)-2-[4-(*(S)*-tetrahydrofuran-3-yloxy)-benzyl]-benzol darstellt und wobei die Menge des SGLT2-Inhibitors 1 bis 25 mg beträgt.

**2.** Pharmazeutische Zusammensetzung nach Anspruch 1, wobei der GLP-1-Rezeptor-agonist ausgewählt ist aus der Gruppe, bestehend aus Exenatid, Exenatid LAR, Liraglutid, Taspoglutid, Semaglutid, Albiglutid, Lixisenatid und Dulaglutid.

**3.** Pharmazeutische Zusammensetzung nach Anspruch 2, wobei der GLP-1-Rezeptoragonist Exenatid oder Exenatid LAR darstellt.

**4.** Pharmazeutische Zusammensetzung nach einem der vorhergehenden Ansprüche, **dadurch gekennzeichnet, dass** die Zusammensetzung für die kombinierte oder gleichzeitige oder aufeinanderfolgende Verwendung des SGLT2-Inhibitors und des GLP-1-Rezeptoragonisten geeignet ist.

**5.** Pharmazeutische Zusammensetzung nach einem der Ansprüche 1 bis 4 zur Verwendung in einem Verfahren zur Behandlung einer Erkrankung oder eines Zustands, ausgewählt aus der Gruppe, bestehend aus Diabetes mellitus, Diabetes mellitus Typ 2 und einer Erkrankung oder einem Zustand, der die Behandlung mit Insulin oder einem GLP-1-Rezeptoragonisten bei einem Patienten erfordert.

**6.** Pharmazeutische Zusammensetzung nach einem der Ansprüche 1 bis 4 zur Verwendung in einem Verfahren zur Behandlung einer Erkrankung oder eines Zustands, ausgewählt aus der Gruppe, bestehend aus

- zur Behandlung von Diabetes mellitus;
- zur Behandlung von Diabetes mellitus, wobei die Behandlung mit Insulin oder einem GLP-1-Rezeptoragonisten erforderlich ist;
- zur Behandlung, Vorbeugung oder Reduzierung des Risikos von Hypoglykämie;
- zur Vorbeugung, zur Verlangsamung des Fortschreitens, zum Verzögern oder Behandeln eines Zustands oder einer Störung, ausgewählt aus der Gruppe, bestehend aus Komplikationen bei Diabetes mellitus;
- zur Vorbeugung, zur Verlangsamung des Fortschreitens, zum Verzögern oder Behandeln einer metabolischen Störung bzw. Stoffwechselstörung, ausgewählt aus der Gruppe, bestehend aus Diabetes mellitus Typ 1, Diabetes mellitus Typ 2, gestörter Glucosetoleranz, gestörtem Nüchternblutzucker, Hyperglykämie, postprandialer Hyperglykämie, Übergewicht, Adipositas, metabolischem Syndrom, Schwangerschaftsdiabetes, Diabetes in Verbindung mit cystischer Fibrose; oder
- zur Verbesserung der glykämischen Kontrolle und/oder zur Reduzierung des Nüchternplasmablutzuckerspiegels, der Nach-Schwangerschaftsplasmaglucose und/oder des glycosylierten Hämoglobins HbA1c, oder
- zur Vorbeugung, Verlangsamung, Verzögerung oder Umkehrung der Progression von gestörter Glucosetoleranz, Insulinresistenz und/oder vom metabolischen Syndrom zu Diabetes mellitus Typ 2; oder
- zur Vorbeugung, zur Verlangsamung des Fortschreitens, zum Verzögern oder Behandeln eines Zustands oder einer Störung, ausgewählt aus der Gruppe, bestehend aus Komplikationen von Diabetes mellitus, wie Katarakten und mikro- und makrovaskulären Erkrankungen, wie Nephropathie, Retinopathie, Neuropathie, Gewebeischämie, diabetischem Fuß, Artheriosklerose, Myokardinfarkt, akutem Koronarsyndrom, instabiler Angina pektoris, stabiler Angina pektoris, Schlaganfall, peripherer arterieller Verschlusskrankheit, Kardiomyopathie, Herzversagen, Herzrhythmusstörungen und vaskulärer Restenose; oder
- zur Reduzierung des Körpergewichts und/oder Körperfetts oder Vorbeugung einer Erhöhung des Körpergewichts und/oder Körperfetts oder Unterstützung einer Reduzierung des Körpergewichts und/oder Körperfetts; oder
- zur Vorbeugung, Verlangsamung, Verzögerung oder Behandlung der Degeneration der Pankreas-beta-Zellen und/oder des Rückgangs der Funktionalität der Pankreas-beta-Zellen und/oder zur Verbesserung und/oder Wiederherstellung der Funktionalität der Pankreas-beta-Zellen und/oder Wiederherstellung der Funktionalität der Pankreas-Insulinsekretion; oder
- zur Vorbeugung, Verlangsamung, Verzögerung oder Behandlung von Erkrankungen oder Zuständen, die einer abnormalen Anreicherung von ektopischem Fett zugeschrieben werden; oder
- zur Aufrechterhaltung und/oder Verbesserung der Insulinsensitivität und/oder zur Behandlung oder Vorbeugung von Hyperinsulinämie und/oder Insulinresistenz;
- zur Vorbeugung, Verlangsamung des Fortschreitens, Verzögerung oder Behandlung von neu-auftretender Diabetes nach Transplantation (NODAT) und/oder post-Transplantations-metabolischem Syndrom (PTMS);
- zur Vorbeugung, Verzögerung oder Reduzierung von mit NODAT und/oder PTMS assoziierten Komplikationen, einschließlich mikro- und makrovaskulären Erkrankungen und Vorfällen, Gewebeabstoßung, Infektion und Tod;
- zur Behandlung von Hyperurikämie und mit Hyperurikämie assoziierten Zuständen;
- zur Behandlung oder Vorbeugung von Nierensteinen;
- zur Behandlung von Hyponatriämie.

**7.** Pharmazeutische Zusammensetzung nach einem der Ansprüche 1 bis 4 zur Verwendung in einem therapeutischen Verfahren zur Gewichtsreduktion, zur Reduktion des Körperfetts, zur Vorbeugung einer Zunahme des Körpergewichts oder zur Verminderung einer Zunahme des Körpergewichts, umfassend eine Verabreichung des GLP-1-Rezeptoragonisten an einen Patienten mit Bedarf hiefür, gefolgt von einer Verabreichung des SGLT2-Inhibitors an den Patienten.

**8.** Pharmazeutische Zusammensetzung nach einem der Ansprüche 1 bis 4 zur Verwendung in einem therapeutischen Verfahren zur Gewichtsreduktion, zur Reduktion des Körperfetts, zur Vorbeugung einer Zunahme des Körpergewichts oder zur Verminderung einer Zunahme des Körpergewichts, wobei die Verabreichung des SGLT2-Inhibitors der Beendigung oder Einstellung einer Behandlung mit dem GLP-1-Rezeptoragonisten folgt.

**9.** Pharmazeutische Zusammensetzung nach einem der Ansprüche 1 bis 4 zur Verwendung in einem Verfahren zur Behandlung einer metabolischen Störung bzw. Stoffwechselstörung, insbesondere von Diabetes mellitus und/oder mit Diabetes mellitus assoziierten Komplikationen, bei einem Patienten, der als übergewichtig oder adipös diagnostiziert wurde, **gekennzeichnet durch** eine erste Therapie, umfassend die Verabreichung des GLP-1-Rezeptoragonisten an den Patienten, gefolgt von einer zweiten Therapie, umfassend die Verabreichung des SGLT2-Inhibitors an den Patienten.

**10.** SGLT2-Inhibitor 1-Chlor-4-(β-D-glucopyranos-1-yl)-2-[4-(*(S)*-tetrahydrofuran-3-yl-oxy)-benzyl]-benzol zur Verwendung in einem therapeutischen Verfahren zur Gewichtsreduktion, zur Reduktion des Körperfetts, zur Vorbeugung einer Zunahme des Körpergewichts oder zur Verminderung einer Zunahme des Körpergewichts, wobei die Verabreichung des SGLT2-Inhibitors eine Beendigung oder Einstellung einer Behandlung mit einem GLP-1-Rezeptoragonisten folgt und wobei der SGLT2-Inhbibitor einmal täglich in einer Menge von 1 bis 25 mg eingesetzt wird.

**11.** SGLT2-Inhibitor 1-Chlor-4-(β-D-glucopyranos-1-yl)-2-[4-*(S)*-tetrahydrofuran-3-yl-oxy)-benzyl]-benzol zur Verwendung in einem Verfahren zur Behandlung einer metabolischen Störung bzw. Stoffwechselstörung, insbesondere von Diabetes mellitus und/oder mit Diabetes mellitus assoziierten Komplikationen, bei einem Patienten, der als übergewichtig oder adipös diagnostiziert wurde, **gekennzeichnet durch** eine erste Therapie, umfassend die Verabreichung eines GLP-1-Rezeptoragonisten an den Patienten, gefolgt von einer zweiten Therapie, umfassend die Verabreichung des SGLT2-Inhibitors an den Patienten und wobei der SGLT2-Inhbibitor einmal täglich in einer Menge von 1 bis 25 mg eingesetzt wird.

## Revendications

1. Composition pharmaceutique comprenant

   (a) un inhibiteur du SGLT2, et
   (b) un agoniste du récepteur du GLP-1,

   sans troisième agent antidiabétique,
   dans laquelle l'inhibiteur du SGLT2 est le 1-chloro-4-(β-D-glucopyranos-1-yl)-2-[4-((*S*)-tétrahydrofuran-3-yloxy)-benzyl]-benzène et dans laquelle la quantité de l'inhibiteur du SGLT2 est de 1 à 25 mg.

2. Composition pharmaceutique selon la revendication 1 dans laquelle l'agoniste du récepteur du GLP-1 est sélectionné dans le groupe consistant en l'exénatide, l'exénatide LAR, le liraglutide, le taspoglutide, le sémaglutide, l'albiglutide, le lixisénatide et le dulaglutide.

3. Composition pharmaceutique selon la revendication 2 dans laquelle l'agoniste du récepteur du GLP-1 est l'exénatide ou l'exénatide LAR.

4. Composition pharmaceutique selon l'une des revendications précédentes **caractérisée en ce que** la composition est appropriée pour une utilisation combinée ou simultanée ou séquentielle de l'inhibiteur du SGLT2 et de l'agoniste du récepteur du GLP-1.

5. Composition pharmaceutique selon l'une des revendications 1 à 4 pour son utilisation dans une méthode de traitement d'une maladie ou d'une affection sélectionnée dans le groupe consistant en le diabète, le diabète de type 2 et une maladie ou une affection qui nécessite un traitement avec de l'insuline ou un agoniste du récepteur du GLP-1 chez un patient.

6. Composition pharmaceutique selon l'une des revendications 1 à 4 pour son utilisation dans une méthode de traitement d'une maladie ou d'une affection sélectionnée dans le groupe consistant en

   - le traitement du diabète ;
   - le traitement du diabète, où le traitement avec de l'insuline ou un agoniste du récepteur du GLP-1 est nécessaire ;
   - le traitement, la prévention ou la réduction du risque d'hypoglycémie ;
   - la prévention, le ralentissement de la progression, le retardement ou le traitement d'une affection ou d'un trouble sélectionné dans le groupe consistant en des complications du diabète ;
   - la prévention, le ralentissement de la progression, le retardement ou le traitement d'un trouble métabolique sélectionné dans le groupe consistant en le diabète de type 1, le diabète de type 2, l'intolérance au glucose, l'altération de la glycémie à jeun, l'hyperglycémie, l'hyperglycémie postprandiale, la surcharge pondérale, l'obésité, le syndrome métabolique, le diabète gestationnel, le diabète associé à la mucoviscidose ; ou
   - l'amélioration du contrôle de la glycémie et/ou la réduction du glucose plasmatique à jeun, du glucose plasmatique postprandial et/ou de l'hémoglobine glycosylée HbA1c ; ou
   - la prévention, le ralentissement, le retardement ou l'inversion de la progression à partir de l'intolérance au glucose, de la résistance à l'insuline et/ou du syndrome métabolique vers le diabète de type 2 ; ou
   - la prévention, le ralentissement de la prévention, le retardement ou le traitement d'une affection ou d'un trouble sélectionné dans le groupe consistant en des complications du diabète telles que la cataracte et les maladies micro- et macro-vasculaires, telles que la néphropathie, la rétinopathie, la neuropathie, l'ischémie tissulaire, le pied diabétique, l'artériosclérose, l'infarctus du myocarde, le syndrome coronarien aigu, l'angine de poitrine instable, l'angine de poitrine stable, l'accident vasculaire cérébral, les maladies occlusives des artères périphériques, la cardiomyopathie, l'insuffisance cardiaque, les troubles du rythme cardiaque et la resténose vasculaire ; ou
   - la réduction du poids corporel et/ou de la graisse corporelle ou la prévention d'une augmentation du poids corporel et/ou de la graisse corporelle ou la facilitation d'une réduction du poids corporel et/ou de la graisse corporelle ; ou
   - la prévention, le ralentissement, le retardement ou le traitement de la dégénérescence des cellules bêta pancréatiques et/ou du déclin de la fonctionnalité des cellules bêta pancréatiques et/ou l'amélioration et/ou la restauration de la fonctionnalité des cellules bêta pancréatiques et/ou la restauration de la fonctionnalité de la sécrétion d'insuline pancréatique ; ou

- la prévention, le ralentissement, le retardement ou le traitement de maladies ou d'affections attribuées à une accumulation anormale de graisse ectopique ; ou
- le maintien et/ou l'amélioration de la sensibilité à l'insuline et/ou le traitement ou la prévention de l'hyperinsulinémie et/ou de la résistance à l'insuline ;
- la prévention, le ralentissement de la progression, le retardement, ou le traitement d'un diabète post-transplantation (NODAT) et/ou d'un syndrome métabolique post-transplantation (PTMS) ;
- la prévention, le retardement ou la réduction des complications associées au NODAT et/ou au PTMS y compris des maladies et des événements micro- et macro-vasculaires, le rejet de greffe, l'infection, et le décès ;
- le traitement de l'hyperuricémie et des affections associées à l'hyperuricémie ;
- le traitement ou la prévention des calculs rénaux ;
- le traitement de l'hyponatrémie.

**7.** Composition pharmaceutique selon l'une des revendications 1 à 4 pour son utilisation dans une méthode thérapeutique de réduction du poids, de réduction de la graisse corporelle, de prévention d'une augmentation du poids corporel ou d'atténuation d'une augmentation du poids corporel comprenant une administration de l'agoniste du récepteur du GLP-1 chez un patient en ayant besoin suivie de l'administration de l'inhibiteur du SGLT2 chez ledit patient.

**8.** Composition pharmaceutique selon l'une des revendications 1 à 4 pour son utilisation dans une méthode thérapeutique de réduction du poids corporel, de réduction de la graisse corporelle, de prévention d'une augmentation du poids corporel ou d'atténuation d'une augmentation du poids corporel, dans laquelle l'administration de l'inhibiteur du SGLT2 suit un arrêt ou une interruption d'un traitement avec l'agoniste du récepteur du GLP-1.

**9.** Composition pharmaceutique selon l'une des revendications 1 à 4 pour son utilisation dans une méthode de traitement d'un trouble métabolique, en particulier du diabète et/ou des complications associées au diabète, chez un patient qui est diagnostiqué comme étant en surpoids ou obèse, **caractérisée par** un premier traitement comprenant l'administration de l'agoniste du récepteur du GLP-1 au patient suivi d'un second traitement comprenant l'administration de l'inhibiteur du SGLT2 au patient.

**10.** Inhibiteur du SGLT2, le 1-chloro-4-(β-D-gluco-pyranos-1-yl)-2-[4-((*S*)-tétrahydrofuran-3-yloxy)-benzyl]-benzène, pour son utilisation dans une méthode thérapeutique de réduction du poids corporel, de réduction de la graisse corporelle, de prévention d'une augmentation du poids corporel ou d'atténuation d'une augmentation du poids corporel, dans laquelle l'administration de l'inhibiteur du SGLT2 suit un arrêt ou une interruption d'un traitement avec un agoniste du récepteur du GLP-1 et dans laquelle l'inhibiteur du SGLT2 est appliqué une fois par jour dans une quantité de 1 à 25 mg.

**11.** Inhibiteur du SGLT2, le 1-chloro-4-(β-D-gluco-pyranos-1-yl)-2-[4-((*S*)-tétrahydrofuran-3-yloxy)-benzyl]-benzène, pour son utilisation dans une méthode de traitement d'un trouble métabolique, en particulier du diabète et/ou des complications associées au diabète, chez un patient qui est diagnostiqué comme étant en surpoids ou obèse, **caractérisée par** un premier traitement comprenant l'administration d'un agoniste du récepteur du GLP-1 au patient suivi d'un second traitement comprenant l'administration de l'inhibiteur du SGLT2 au patient et dans laquelle l'inhibiteur du SGLT2 est appliqué une fois par jour dans une quantité de 1 à 25 mg.

Figure 1

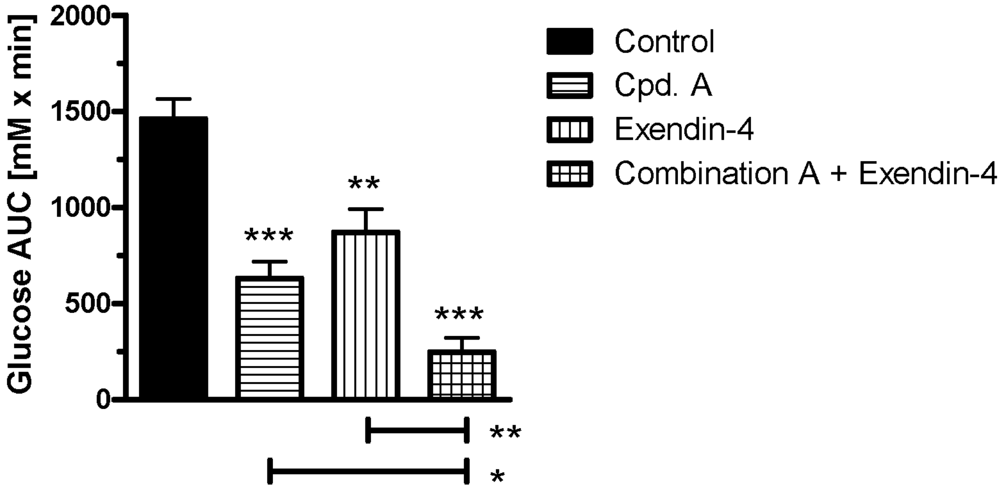
Glucose AUC [mM x min]
2000
1500
1000
500
0
***
**
***
**
*
Control
Cpd. A
Exendin-4
Combination A + Exendin-4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0127128 A
- WO 03099836 A
- WO 2005092877 A
- WO 2006034489 A
- WO 2006064033 A
- WO 2006117359 A
- WO 2006117360 A
- WO 2007025943 A
- WO 2007028814 A
- WO 2007031548 A
- WO 2007093610 A
- WO 2007128749 A
- WO 2008049923 A
- WO 2008055870 A
- WO 2008055940 A
- WO 2006124529 A
- WO 2009020802 A
- WO 2010092126 A
- WO 2010092124 A
- WO 2006120208 A


### Non-patent literature cited in the description


- **FORD ES et al.** *JAMA,* 2002, vol. 287, 356-9
- **KATSUKI A et al.** *Diabetes Care,* 2001, vol. 24, 362-5
- **MATTHEWS et al.** *Diabetologia,* 1985, vol. 28, 412-19
- **FORST et al.** *Diabetes,* 2003, vol. 52 (1), A459
- **GALVIN P et al.** *Diabet Med,* 1992, vol. 9, 921-8
- **STUMVOLL et al.** *Eur J Clin Invest,* 2001, vol. 31, 380-81
- **J. B. MEIGS et al.** *Diabetes,* 2003, vol. 52, 1475-1484
- The Prevention or Delay of Type 2 Diabetes. Diabetes Care. American Diabetes Association and the National Institute of Diabetes and Digestive and Kidney Diseases, 2002, vol. 25, 742-749
- **LAAKSONEN DE et al.** *Am J Epidemiol,* 2002, vol. 156, 1070-7
- Executive Summary of the Third Report of the National Cholesterol Education Program (NCEP) Expert Panel on Detection, Evaluation, and Treatment of High Blood Cholesterol in Adults (Adult Treatment Panel I I I). *JAMA: Journal of the American Medical Association,* 2001, vol. 285, 2486-2497
- **LAAKSONEN DE et al.** *Am J Epidemiol.,* 2002, vol. 156, 1070-7